(19) Europäisches Patentamt
European Patent Office
Office européen des brevets

(11) Veröffentlichungsnummer: **0 334 096**
**A2**

(12) # EUROPÄISCHE PATENTANMELDUNG

(21) Anmeldenummer: 89103978.6

(51) Int. Cl.[4]: C07C 49/84 , C07C 45/46

(22) Anmeldetag: 07.03.89

(30) Priorität: 19.03.88 DE 3809260
14.06.88 DE 3820193

(43) Veröffentlichungstag der Anmeldung:
27.09.89 Patentblatt 89/39

(84) Benannte Vertragsstaaten:
CH DE ES FR GB IT LI NL

(71) Anmelder: BAYER AG
D-5090 Leverkusen 1 Bayerwerk(DE)

(72) Erfinder: Botta, Artur, Dr.
Bodelschwinghstrasse 119
D-4150 Krefeld(DE)
Erfinder: Buysch, Hans-Josef, Dr.
Brandenburger Strasse 28
D-4150 Krefeld(DE)
Erfinder: Puppe, Lothar, Dr.
Am Weiher 10a
D-5093 Burscheid(DE)
Erfinder: Arlt, Dieter, Prof. Dr.
Rybnikerstrasse 2
D-5000 Köln 80(DE)

(54) **Verfahren zur Herstellung von in 4-Stellung veretherten Phenylketonen.**

(57) In 4-Stellung veretherte Phenylketone können durch Umsetzung von aromatischen Ethern mit freier 4-Stellung mit Acylierungsmitteln hergestellt werden, wobei man in Gegenwart von Zeolith-Katalysatoren arbeitet, die Porenweiten von mindestens 5 Å haben.

EP 0 334 096 A2

## Verfahren zur Herstellung von in 4-Stellung veretherten Phenylketonen

Die Erfindung betrifft ein Verfahren zur Herstellung von in 4-Stellung veretherten Phenylketonen durch Umsetzung von aromatischen Ethern, die eine freie 4-Stellung haben, mit üblichen Acylierungsmitteln an mittel- oder weitporigen Zeolithen, die eine Porenweite von mindestens 5 Å haben.

Ethergruppenhaltige aromatische Ketone, beispielsweise 4-Methoxy-acetophenon oder 4-Methoxy-propiophenon, beanspruchen starkes Interesse in der Riechstoffindustrie oder als Zwischenprodukte für andere Synthesen. Die übliche Methode zur Darstellung von aromatischen Ketonen ist die homogene Friedel-Crafts-Acylierung von aromatischen Kohlenwasserstoffen mit Carbonsäurederivaten; in dieser Form wird die Acylierung auch technisch praktiziert. Hierzu sind bekanntlich mindestens stöchiometrische, meist jedoch überschüssige Mengen des Katalysators erforderlich, wie Lewis-Säuren ($AlCl_3$, $FeCl_3$, $BF_3$, $ZnCl_2$, $TiCl_4$) bzw. Protonensäuren (Polyphosphorsäure, HF). Verwiesen wird beispielsweise auf die Monographie von G. A. Olah, "Friedel-Crafts and related reactions, Wiley-Interscience, New York, Vol. I-IV (1963-1964) oder auf DE-OS 35 19 009. Die Problematik der technisch durchgeführten Friedel-Crafts-Katalyse, nämlich hohe Kosten durch erhöhte Korrosion sowie erheblicher Aufwand in der Handhabung, Abtrennung und Entsorgung des letztendlich verbrauchten Katalysators, ist hinreichend bekannt.

Im speziellen Fall des Einsatzes von ethergruppenhaltigen Aromaten ist darüber hinaus auch mit einer Etherspaltung bzw. einer Umlagerung des am Ethersauerstoff gebundenen Kohlenwasserstoffrests in den Kern zu rechnen.

Es wurde nun ein Verfahren zur Herstellung von in 4-Stellung veretherten Phenylketonen der Formel

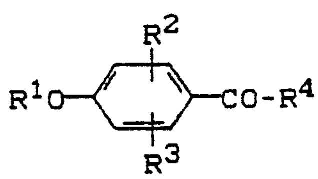

(I)

durch Umsetzung von aromatischen Ethern der Formel

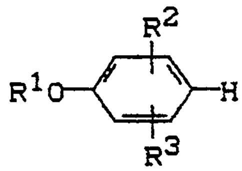

(II)

mit Acylierungsmitteln der Formel

$X\text{-}CO\text{-}R^4$ (III),

wobei in den Formeln

$R^1$ für $C_1$-$C_{12}$-Alkyl, $C_2$-$C_{12}$-Alkenyl, $C_3$-$C_7$-Cycloalkyl oder $C_6$-$C_{10}$-Aryl steht,

$R^2$ und $R^3$ unabhängig voneinander Wasserstoff, Fluor, Chlor, Brom, $C_1$-$C_4$-Alkyl oder $C_3$-$C_7$-Cycloalkyl bedeuten,

$R^4$ $C_1$-$C_{16}$-Alkyl, $C_2$-$C_{16}$-Alkenyl, $C_3$-$C_7$-Cycloalkyl, $C_7$-$C_{12}$-Aralkyl, $C_8$-$C_{12}$-Aralkenyl oder $C_6$-$C_{12}$-Aryl bedeutet und

X für Chlor, Brom, $-OCOR^4$, $C_1$-$C_2$-Alkoxy, Hydroxyl, Amino, NH-$C_1$-$C_4$-Alkyl oder N($C_1$-$C_4$-Alkyl)$_2$ steht,

gefunden, das dadurch gekennzeichnet ist, daß die Umsetzung in Gegenwart von Zeolith-Katalysatoren der Formel

$M_{m/z}[mMe^1O_2 \bullet nMe^2O_2] \bullet q\ H_2O$ (IV), worin

M ein austauschbares Kation bedeutet,

z die Wertigkeit des Kations bedeutet,

$Me^1$ und $Me^2$ die Elemente des anionischen Gerüstes darstellen,

n/m das Verhältnis der Elemente bedeutet und Werte von 1-3000, bevorzugt 1-2000, annimmt und

q die Menge des sorbierten Wassers bedeutet,

wobei die Zeolithe Porenweiten von mindestens 5 Å haben,

durchgeführt wird.

$R^1$ kann $C_1$-$C_{12}$-Alkyl bedeuten, bevorzugt $C_1$-$C_4$-Alkyl, besonders bevorzugt $C_1$-$C_2$-Alkyl, ganz beson-

ders bevorzugt Methyl. Beispiele sind außer Methyl Ethyl, Propyl, Isopropyl, Butyl, Isobutyl, die isomeren Amyle, Hexyle, Octyle, Decyle, Dodecyle. $R^1$ kann weiterhin $C_2$-$C_{12}$-Alkenyl bedeuten, bevorzugt $C_2$-$C_4$-Alkenyl, wie Vinyl, Propenyl, Butenyl, die isomeren Amylene, Hexene, Octene, Decene, Dodecene. $R^1$ kann weiterhin $C_6$-$C_{10}$-Aryl bedeuten, wie Phenyl oder Naphthyl, bevorzugt Phenyl. $R^1$ kann weiterhin $C_3$-$C_7$-Cycloalkyl bedeuten, wie Cyclopropyl, Cyclobutyl, Cyclopentyl, Methyl-cyclopentyl, Cyclohexyl, Methyl-cyclohexyl oder Cycloheptyl. $R^2$ und $R^3$ können unabhängig voneinander $C_1$-$C_4$-Alkyl, bevorzugt $C_1$-$C_2$-Alkyl, besonders bevorzugt Methyl bedeuten. $R^2$ und $R^3$ können weiterhin unabhängig voneinander $C_3$-$C_7$-Cycloalkyl des obigen Umfangs bedeuten, bevorzugt Cyclopropyl, Cyclopentyl und Cyclohexyl. $R^4$ kann $C_1$-$C_{16}$-Alkyl, bevorzugt $C_1$-$C_{12}$-Alkyl, besonders bevorzugt $C_1$-$C_4$-Alkyl, beispielsweise der obengenannten Art, bedeuten. $R^4$ kann weiterhin $C_2$-$C_{16}$-Alkenyl, bevorzugt $C_2$-$C_4$-Alkenyl, beispielsweise der obengenannten Art, bedeuten. $R^4$ kann weiterhin $C_3$-$C_7$-Cycloalkyl, beispielsweise der obengenannten Art, bedeuten. $R^4$ kann weiterhin $C_7$-$C_{12}$-Aralkyl, bevorzugt $C_7$-$C_9$-Aralkyl, bedeuten, beispielsweise Benzyl, 1-Phenyl-ethyl, 2-Phenyl-ethyl, Naphthyl-methyl, Naphthyl-ethyl; besonders bevorzugt ist Benzyl. $R^4$ kann weiterhin $C_6$-$C_{12}$-Aryl bedeuten, wie Phenyl, Naphthyl oder Biphenyl, bevorzugt Phenyl. $R^4$ kann weiterhin Aralkenyl mit $C_8$-$C_{12}$ bedeuten, bevorzugt Styryl.

Die genannten Reste können ihrerseits durch Fluor, Chlor, Brom, $C_1$-$C_4$-Alkyl, $C_1$-$C_4$-Alkoxy bzw. $C_1$-$C_4$-Alkylthio substituiert sein. $R^4$ kann weiterhin durch eine zweite Carboxylgruppe oder deren funktionelles Derivat substituiert sein.

Als Acylierungsmittel kommen die von Formel (III) abgeleiteten Säuren, ihre Halogenide, ihre Ester, ihre Anhydride, ihre substituierten oder nicht-substituierten Amide in Frage; besonders kommen die Anhydride und die Chloride in Frage.

Ein repräsentatives Beispiel für die erfindungsgemäße Umsetzung ist die Reaktion von Anisol mit Essigsäureanhydrid, die sich durch folgendes Formelschema verdeutlichen läßt:

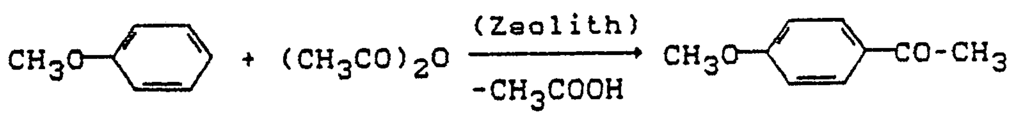


Die genannten Nachteile der bekannten Verfahren werden durch das erfindungsgemäße Verfahren vermieden. Es ist zwar bereits bekannt, niederalkylsubstituierte Benzole ohne Hydroxylgruppen in Gegenwart von Zeolithen mit Carbonsäuren oder Carbonsäurederivaten, vorzugsweise in der Gasphase, zu aromatischen Ketonen umzusetzen. Dabei werden mit Essigsäure an Pentasilen (EP 239 383) oder an Zeolith SE-Y (DE-OS 26 16 583) Umsätze nur in einer Größenordnung von 5 % erzielt. Höhere Umsätze werden nach FR 2.592.039 bzw. J. Org. Chem. 51 (1986), 2128-2130 nur mit längerkettigen Carbonsäuren erreicht. Andererseits wird in US 4.652.683, US 4.668.826 und EP 227 331 berichtet, Phenol mit Carbonsäuren in der Gasphase an Silicalit oder H-ZSM 5 zu bevorzugt 2-Hydroxyphenyl-niederalkylketonen zu acylieren; teilweise treten hierbei jedoch Nebenprodukte wie Phenylacetat oder Folgeprodukte, beispielsweise Heterocyclen, wie 2-Methyl-chromon und 4-Methyl-cumarin auf. Gemische dieser Art aus Estern, Ketonen und Heterocyclen sind auch das Ergebnis der Umsetzung von Phenolen mit Essigsäureanhydrid in der Gasphase, beispielsweise an Mordenit (Kin. Katal. 23 (1982/2), 417-420; zitiert nach C.A. 97, 72 012 f).

Im Hinblick auf den genannten Stand der Technik war es nicht zu erwarten, daß beim erfindungsgemäßen Verfahren auch mit kurzkettigen Carbonsäurederivaten, wie Essigsäureanhydrid, ausgezeichnete Umsätze von 75 % oder mehr erzielt werden können, daß weiterhin mit hoher Selektivität von 98-100 % 4-Alkoxyphenyl-ketone statt der 2-Alkoxy-Isomeren erhalten werden und daß schließlich unter den Reaktionsbedingungen die Alkoxyaromaten an Zeolithen, wenn sie in ihrer sauren Form eingesetzt werden, keine Etherspaltungs- und/oder Umlagerungsreaktionen zu Phenolen oder Kresolen eingehen.

Aromatische Ether für das erfindungsgemäße Verfahren sind beispielsweise die folgenden: Anisol, o- und m-Methylanisol, o- und m-Chlor- oder Bromanisol, o-Ethylanisol, Phenetol, o- und m-Methylphenetol, m-Chlorphenetol, Propoxy-, Butoxy-, Isobutoxy-, Amyloxy-, Octyloxy-, Decyloxy-, Lauryloxy-, Cyclohexyloxy-, Benzyloxy-, Hexenyloxy-benzol, -3-chlorbenzol, -3-methylbenzol, -2-ethylbenzol, Diphenylether, 2-Chlordiphenylether, -3-methyl-diphenylether.

Acylierungsmittel für das erfindungsgemäße Verfahren sind beispielsweise die folgenden: Essigsäure, Essigsäurechlorid, Essigsäurebromid, Essigsäureanhydrid, Essigsäuremethylester, Essigsäureamid, Propionsäure, Propionsäurechlorid, Propionsäureanhydrid, Buttersäure, Buttersäurechlorid, Buttersäureanhydrid, Isobuttersäure, Isobuttersäurechlorid, Isobuttersäureanhydrid, Pivalinsäurechlorid, Pivalinsäureanhydrid, Valeriansäure, Valeriansäurechlorid, Valeriansäureanhydrid, Capronsäurechlorid, Isooctansäurechlorid,

Laurinsäurechlorid, Chloressigsäurechlorid, Dichloressigsäurechlorid, Dichlorfluoressigsäurechlorid, Chlorbuttersäurechlorid, Methoxyessigsäureanhydrid, Butylmercaptoessigsäure, Butylmercaptoessigsäurechlorid, Acrylsäure, Methacrylsäurechlorid, Cyclopropylcarbonsäurechlorid, Cyclohexancarbonsäurechlorid, Phenylessigsäurechlorid, Phenylessigsäureanhydrid, Dihydrozimtsäurechlorid, Zimtsäurechlorid, Benzoesäurechlorid, Benzoesäureanhydrid, Benzoesäuremethylester, o-, m-, p-Fluor-, -Chlor-, -Brom- und -Iod-benzoesäurechlorid, o-, m-, p-Methyl-, -Isopropyl-, -Methoxybenzoesäurechlorid, Dichlor-, Dimethyl-, Methoxy-methylbenzoesäurechlorid, Chlor-methylbenzoesäurechlorid, Malonsäureanhydrid, Dimethylmalonsäuredichlorid, Bernsteinsäureanhydrid, Glutarsäureanhydrid, Phthalsäureanhydrid, Phthalsäuredichlorid, Tetrahydrophthalsäureanhydrid.

Die Einsatzmenge an Acylierungsmittel kann im erfindungsgemäßen Verfahren in weiten Grenzen variiert werden. Im allgemeinen setzt man das Acylierungsmittel im stöchiometrischen Verhältnis zum aromatischen Ether ein. Es kann aber auch von Vorteil sein, eine Komponente im Überschuß zu verwenden, beispielsweise den Ether mit 0,2-5 Mol, vorzugsweise 0,5-2 Mol, bezogen auf das Acylierungsmittel.

Das erfindungsgemäße Verfahren wird in Gegenwart von Zeolith-Katalysatoren der Formel (IV) durchgeführt. Zeolithe sind von ihrer Grundstruktur her kristalline Alumosilikate, die aus einem Netzwerk von $SiO_4$- bzw. $AlO_4$-Tetraedern aufgebaut sind. Die einzelnen Tetraeder sind mit Sauerstoffbrücken über die Ecken untereinander verknüpft und bilden ein räumliches Netzwerk, das gleichmäßig von Kanälen und Hohlräumen durchzogen ist. Die einzelnen Zeolith-Strukturen unterscheiden sich durch die Anordnung und Größe der Kanäle und Hohlräume sowie durch ihre Zusammensetzung. Als Ausgleich für die negative Ladung des Gitters sind austauschbare Kationen eingelagert. Erfindungsgemäß einsetzbare Zeolithe fallen unter die Formel (IV). Hierin bildet q $H_2O$ eine sorbierte Wasserphase, die reversibel entfernbar ist, ohne daß das Gerüst seine Struktur verliert. In (IV) ist $Me^1$ im allgemeinen Aluminium, das aber durch andere Elemente, wie B, Ga, In, Fe, Cr, V, As, Sb oder Be teilweise ersetzt sein kann. Weiterhin ist in (IV) $Me^2$ hauptsächlich Silicium, das jedoch durch andere 4-wertige Elemente ersetzt sein kann, wie beispielsweise Ge, Ti, Zr oder Hf.

Eine ausführliche Darstellung von Zeolithen ist beispielsweise in der Monographie von D. W. Breck "Zeolite Molecular Sieves, Structure, Chemistry, and Use", J. Wiley and Sons, New York, 1974 gegeben.

In bevorzugter Weise kommen Zeolithe der folgenden Strukturtypen für das erfindungsgemäße Verfahren in Frage: Faujasit, L, Mordenit, Mazzit, Offretit, Gmelinit, Cancrinit, ZSM 12, ZSM 25, Zeolith $\beta$, Ferrierit, ZSM 5, ZSM 11, Heulandit, ZSM 22, ZSM 23, ZSM 48, ZSM 43, ZSM 35, PSH-3, Zeolith $\rho$, ZSM 38, CSZ-1, ZSM 3, ZSM 20, Chabasit, besonders bevorzugt Zeolith L, Mordenit, ZSM 5, ZSM 11, ZSM 12, ZSM 23 und Offretit. Ganz besonders bevorzugt sind die Zeolithtypen Mordenit, L, ZSM 5 und ZSM 11.

Erfindungsgemäß einsetzbare Zeolithe haben Porenweiten von mindestens 5 Å, beispielsweise solche im Bereich von 5-9 Å, bevorzugt im Bereich von 5-7 Å.

Als austauschbare Kationen können die Zeolithe z.B. solche des Li, Na, K, Mg, Cu, Ca, Zn, seltene Erdmetalle, Ti, Zr, Sn (IV), Cr (III), Fe (II), Mn (II), Co, Ni und andere enthalten. Erfindungsgemäß bevorzugt sind solche Zeolithe, bei denen zumindest ein Teil der Metallionen gegen Wasserstoffionen ausgetauscht wurde, bevorzugt 50 bis 100%, besonders bevorzugt 80 bis 100% aller ursprünglich vorhandenen Metallkationen. Die sauren $H^+$-Formen der Zeolithe werden bevorzugt dadurch hergestellt, daß man Metall gegen Ammoniumionen austauscht und anschließend kalziniert. Eine weitere Möglichkeit besteht darin, bei Zeolithen, die einen n/m-Wert von 5 oder größer aufweisen, den Protonenaustausch mit Mineralsäuren vorzunehmen. Weiterhin bevorzugt für das erfindungsgemäße Verfahren sind daher die H-Formen der Zeolithe vom Strukturtyp Mordenit, ZSM 5, ZSM 11, Zeolith L, ZSM 12, ZSM 23 und Offretit.

Der Zeolith-Katalysator kann in einer Menge von 1-100 Gew.-%, bevorzugt 5-50 Gew.-%, besonders bevorzugt 10 bis 30 Gew.-%, bezogen auf das Gesamtgewicht der umzusetzenden organischen Reaktionspartner, eingesetzt werden.

Der Zeolith-Katalysator ist mehrfach für das erfindungsgemäße Verfahren wiederverwendbar. Sollten nach mehrfacher Wiederverwendung Aktivitätsverluste eintreten, kann er in einer dem Fachmann geläufigen Weise regeneriert werden, beispielsweise durch Waschen, Säurebehandlung und Kalzinieren.

Die Acylierungsreaktion kann im allgemeinen in der Schmelze durchgeführt werden, sofern es die Schmelz-bzw. Siedepunktsverhältnisse der Reaktionspartner zulassen. Selbstverständlich können aber auch Lösungsmittel mitverwendet werden. Als Lösungsmittel kommen solche in Frage, die unter den Reaktionsbedingungen gegenüber den eingesetzten Zeolithen und Acylierungsmitteln inert sind, beispielsweise Kohlenwasserstoffe oder Halogenkohlenwasserstoffe, wie Petrolether, Cyclohexan oder Dichlorbenzol, Ether, wie Tetrahydrofuran oder Dioxan. Diese Aufzählung ist keineswegs erschöpfend.

Das erfindungsgemäße Verfahren kann kontinuierlich oder diskontinuierlich, bei normalem, erniedrigtem oder erhöhtem Druck durchgeführt werden. Fernerhin kann die erfindungsgemäße Acylierung in der Gasphase oder in der Flüssigkeitsphase durchgeführt werden. Für die Durchführung in der Gasphase wird

der Zeolith-Katalysator in stückiger Form in einem Reaktionsrohr angebracht. Für die Flüssigphase wird der Zeolith-Katalysator im allgemeinen in pulverisierter Form verwendet. Die bevorzugte Ausführungsform ist die in der Flüssigphase; sie kann in der Sumpf-, Riesel- oder Suspensionsfahrweise durchgeführt werden. Die obengenannte, an sich unkritische Anwendung von Druck wird für die bevorzugte Durchführung in der flüssigen Phase lediglich dazu benutzt, um niedrigsiedende Reaktionspartner in dieser bevorzugten flüssigen Phase zu halten.

Das erfindungsgemäße Verfahren wird bei einer Temperatur von 25-400° C durchgeführt. Für die Gasphase wird der höhere Temperaturbereich, beispielsweise 140-400° C angewandt, für die Flüssigphase wird der niedrigere Temperaturbereich, beispielsweise 25-250° C, bevorzugt 120 bis 200° C angewandt.

Die Isolierung und Reinigung der acylierten Endprodukte erfolgt nach beendeter Umsetzung durch bekannte gebräuchliche Techniken, beispielsweise nach vorheriger Abtrennung des Katalysators durch Destillation und/oder Umkristallisation und/oder chromatographische Methoden.

Nicht verbrauchte Ausgangsprodukte können, ebenso wie der zurückgewonnene Katalysator, wieder in das erfindungsgemäße Verfahren zurückgeführt werden.

Der Druck für die flüssige Phase kann der sich einstellende Eigendruck in einem Autoklaven sein; er kann jedoch auch durch einen additiven Inertgasdruck von beispielsweise zusätzlichen 0,01-50 bar verstärkt werden. Inertgase sind beispielsweise $N_2$, He, Ar, oder $CO_2$.

## Beispiele

In den Beispielen wurden folgende Zeolithe eingesetzt:

| Beispiel | Typ | $SiO_2/Al_2O_3$ |
|---|---|---|
| 1 | H-Mordenit | 25 |
| 2 | H-ZSM 5 | 110 |
| 3 | H-Zeolith L | 6 |
| 4 | H-Zeolith L | 7,5 |
| 5 | H-Mordenit | 25 |
| 6 | H-Mordenit | 16 |
| 7 | H-ZSM 11 | 65 |
| 8 | H-Offretit/Erionit | 6 |
| 9 | H-ZSM 5 | 110 |
| 10 | H-ZSM 5 | 110 |
| 11 | H-ZSM 5 | 110 |
| 12 | H-ZSM 5 | 110 |
| 13 | H-ZSM 5 | 110 |
| 14 | H-Mordenit | 16 |
| 15 | H-Mordenit | 16 |
| Vergleich | ohne Katalysator | |

### Beispiel 1

216,3 g (2 Mol) Anisol, 102,1 g (1 Mol) Essigsäureanhydrid und 40 g Zeolithpulver wurden in einem 1 l-Autoklaven, der mit einem Teflon-Hemd ausgekleidet war, unter Rühren und 20 bar Stickstoffdruck auf 160° C erhitzt und 3 h bei dieser Temperatur gehalten. Nach Erkalten wurde die Zusammensetzung gaschromatographisch bestimmt. Das Ergebnis ist in Tabelle 1 aufgeführt.

### Beispiele 2 bis 8

Eine Mischung aus 54 g (0,5 Mol) Anisol, 25,5 g (0,25 Mol) Essigsäureanhydrid und 8 g aktiviertem Zeolithpulver wurde zum Rückfluß erhitzt (140° ) und in bestimmten Zeitabständen wurden Proben zur gaschromatographischen Bestimmung entnommen, Die Ergebnisse gehen aus Tabelle 1 hervor.

Vergleichsbeispiel

Das Beispiel 2 wurde ohne Katalysator wiederholt; die Ergebnisse gehen ebenfalls aus Tabelle 1 hervor.

Beispiel 9

Bei gleicher Verfahrensweise wie in Beispiel 2 wurden 37,9 g (0,35 Mol) Anisol mit 35,7 g (0,35 Mol) Essigsäureanhydrid in Gegenwart von 8 g Zeolithpulver umgesetzt. Ergebnisse in Tab. 1.

Beispiel 10

Analog Beispiel 2 wurden 32,5 g (0,3 Mol) Anisol mit 45,9 g (0,45 Mol) Essigsäureanhydrid in Gegenwart von 8 g Zeolithpulver umgesetzt. Ergebnisse in Tab. 1.

Beispiel 11

Zu einer Suspension von 20 g Zeolithpulver in 108 g (1 Mol) Anisol wurden bei Rückflußtemperatur von 140 °C im Verlauf von 2 h 39,3 g (0,5 Mol) Acetylchlorid zugetropft, wobei die Temperatur zeitweise auf 80 °C absank. Man rührte noch weitere 8 h bei Rückflußtemperatur nach und bestimmte die Zusammensetzung gaschromatographisch. Tabelle 2 enthält die Ergebnisse.

Beispiel 12

Eine Mischung von 54,0 g (0,5 Mol) Anisol, 19,8 g (0,25 Mol) Acetylchlorid und 15 g Zeolithpulver wurde unter Rühren zum Rückfluß erhitzt, wobei die Temperatur im Verlaufe von 10 h auf 90 °C anstieg. Das Ergebnis der gaschromatographischen Analyse geht aus Tabelle 2 hervor.

Beispiel 13

Analog Beispiel 11 wurden 54 g (0,5 Mol) Anisol, 39,5 g (0,5 Mol) Acetylchlorid und 15 g Zeolithpulver miteinander umgesetzt. Das Ergebnis der gaschromatographischen Bestimmung zeigt Tabelle 2.

Beispiel 14

Analog der Verfahrensweise des Beispiels 2 wurden 27 g (0,25 Mol) Anisol, 16,25 g (0,125 Mol) Propionsäurean hydrid und 2,95 g Zeolithpulver bei Rückflußtemperatur von 150 °C miteinander umgesetzt. Die gaschromatographischen Befunde werden in Tabelle 3 dargestellt.

Beispiel 15

Bei gleicher Verfahrensweise wie in Beispiel 12 wurden 27 g (0,25 Mol) Anisol, ,11,6 g (0,125 Mol) Propionsäurechlorid und 3 g Zeolithpulver miteinander umgesetzt, wobei die Rückflußtemperatur im Verlauf von 5 h von 105 auf 157 °C anstieg. Das Ergebnis der gaschromatographischen Analyse geht aus Tabelle 3 hervor.

Tabelle 1

| Acylierung von Anisol mit Essigsäureanhydrid ($Ac_2O$) (Produktzusammensetzung in Massen-%) 2-MAP = 2-Methoxy-acetophenon; 4-MAP = 4-Methoxyacetophenon; U = Umsatz; S = Selektivität zu 4-MAP. | | | | | | | |
|---|---|---|---|---|---|---|---|
| Bsp. | Reakt.-zeit (h) | $Ac_2O$ | Anisol | U (%) | 2-MAP | 4-MAP | S |
| 1 | 3 | 1,3 | 41,7 | 79,5 | 0,40 | 36,5 | 98,0 |
| 2 | 7,5 | 6,4 | 47,9 | 63,5 | - | 28,9 | 96,5 |
| | 12,5 | 1,3 | 47,5 | 65,0 | - | 30,8 | 94,6 |
| 3 | 1 | 14,1 | 58,0 | 35,5 | 0,92 | 18,2 | 95,2 |
| | 3 | 8,3 | 53,0 | 51,0 | 1,47 | 24,8 | 94,5 |
| | 6 | 5,4 | 50,7 | 57,5 | 1,75 | 28,0 | 94,1 |
| 4 | 1,25 | 13,7 | 60,4 | 30,5 | - | 16,3 | 100 |
| | 3,25 | 10,5 | 58,7 | 36,0 | - | 19,2 | 100 |
| | 5,75 | 8,3 | 57,4 | 40,0 | 0,18 | 20,9 | 99,2 |
| 5 | 1 | 6,4 | 51,3 | 57,8 | - | 28,9 | 100 |
| | 3 | 3,2 | 47,1 | 70,5 | - | 34,9 | 100 |
| | 5 | 2,6 | 45,4 | 76,0 | - | 37,1 | 100 |
| 6 | 1 | 9,3 | 55,6 | 45,0 | - | 22,9 | 100 |
| | 3 | 8,3 | 54,4 | 48,5 | - | 24,7 | 100 |
| | 5,5 | 5,8 | 54,3 | 50,0 | - | 25,5 | 100 |
| 7 | 0,75 | 16,6 | 61,3 | 27,0 | - | 14,5 | 100 |
| | 2,75 | 10,2 | 56,4 | 43,0 | - | 21,8 | 100 |
| | 6,75 | 6,1 | 54,8 | 48,8 | - | 24,7 | 99,3 |
| 8 | 1 | 14,4 | 65,0 | 16,5 | - | 10,7 | 100 |
| | 3 | 15,7 | 63,5 | 21,5 | - | 12,1 | 100 |
| | 5,5 | 14,1 | 63,4 | 22,5 | - | 12,7 | 100 |
| Vergl. | 1-8 | 32,07 | 67,9 | 0 | - | - | 0 |
| 9 | 1 | 18,6 | 27,4 | 48,0 | - | 32,5 | 99,6 |
| | 10 | 17,7 | 24,3 | 53,5 | - | 37,3 | 95,7 |
| 10 | 1,75 | 40,9 | 16,2 | 61,5 | - | 35,2 | 99,3 |
| | 4,25 | 37,1 | 14,6 | 66,0 | - | 37,7 | 99,0 |
| | 6,5 | 27,9 | 14,2 | 66,5 | - | 40,8 | 100 |

Tabelle 2

| Acylierung von Anisol mit Acetylchlorid im Molverhältnis Anisol : Acetylchlorid = 2:1 (Beispiele 11 und 12) bzw. 1:1 (Beispiel 13). Weitere Angaben wie in Tabelle 1 | | | | | | |
|---|---|---|---|---|---|---|
| Bsp. | Reaktionszeit(h); | -temp.(°C) | Anisol | 2-MAP | 4-MAP | S |
| 11 | 4<br>10 | 140<br>bis auf<br>80 | 57,3<br>60,6 | -<br>- | 22,5<br>22,4 | 96,8<br>96,8 |
| 12 | 1,5<br>6<br>9,75 | 35<br>bis<br>auf 90 | 75,2<br>75,6<br>64,7 | 0,46<br>0,30<br>0,83 | 2,4<br>2,2<br>14,3 | 72,2<br>73,9<br>90,6 |
| 13 | 1<br>6 | 140<br>bis auf<br>80 | 62,2<br>61,2 | 1,2<br>1,2 | 13,3<br>14,1 | 91,7<br>92,2 |

Tabelle 3: Acylierung von Anisol mit $C_2H_5CO$-X im Molverhältnis 2:1 (X = OCO-$C_2H_5$ in Beispiel 14; X = Cl in Beispiel 15). 2-MPP bzw. 4-MPP = 2- bzw. 4-Methoxypropiophenon. Weitere Angaben wie in Tabelle 1 bzw. 2.

| Bsp. | Reaktions-zeit(h); | -temp.(°C) | Anisol | $C_2H_5COX$ | 2-MPP | 4-MPP | S |
|---|---|---|---|---|---|---|---|
| 14 | 2 | 150 | 38,1 | 5,1 | 0,55 | 37,5 | 98,6 |
| | 4 | 150 | 33,7 | 0,6 | 0,64 | 43,0 | 98,5 |
| | 6 | 150 | 33,3 | 0,2 | 0,74 | 42,6 | 98,3 |
| (Ausgangsgemisch | | | 62,4 | 37,6) | | | |
| 15 | 1 | 150-157 | 58,1 | 1,4 | 0,28 | 22,9 | 98,8 |
| | 2 | 150-157 | 53,4 | 1,4 | 0,37 | 28,2 | 98,7 |
| | 3 | 150-157 | 46,7 | 1,1 | 0,37 | 34,0 | 98,9 |
| | 4 | 150-157 | 42,6 | 0,5 | 0,46 | 40,4 | 98,9 |

## Ansprüche

1. Verfahren zur Herstellung von in 4-Stellung veretherten Phenylketonen der Formel

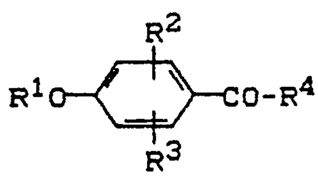

durch Umsetzung von aromatischen Ethern der Formel

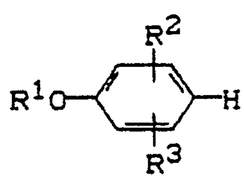

mit Acylierungsmitteln der Formel
X-CO-$R^4$ ,
wobei in den Formeln
$R^1$ für $C_1$-$C_{12}$-Alkyl, $C_2$-$C_{12}$-Alkenyl, $C_3$-$C_7$-Cycloalkyl oder $C_6$-$C_{10}$-Aryl steht,
$R^2$ und $R^3$ unabhängig voneinander Wasserstoff, Fluor, Chlor, Brom, $C_1$-$C_4$-Alkyl oder $C_3$-$C_7$-Cycloalkyl bedeuten,
$R^4$ $C_1$-$C_{16}$-Alkyl, $C_2$-$C_{16}$-Alkenyl, $C_3$-$C_7$-Cycloalkyl, $C_7$-$C_{12}$-Aralkyl, $C_8$-$C_{12}$-Aralkenyl oder $C_6$-$C_{12}$-Aryl bedeutet und
X für Chlor, Brom, -OCOR$^4$, $C_1$-$C_4$-Alkoxy, Hydroxyl, Amino, NH-$C_1$-$C_4$-Alkyl oder N($C_1$-$C_4$-Alkyl)$_2$ steht,
dadurch gekennzeichnet, daß die Umsetzung in Gegenwart von Zeolith-Katalysatoren der Formel
$M_{m/z}[mMe^1O_2 \bullet nMe^2O_2] \bullet q\ H_2O$ ,
worin
M ein austauschbares Kation bedeutet,
z die Wertigkeit des Kations bedeutet,

$Me^1$ und $Me^2$ die Elemente des anionischen Gerüstes darstellen,
n/m das Verhältnis der Elemente bedeutet und Werte von 1-3000, bevorzugt 1 bis 2000, annimmt und
q die Menge des sorbierten Wassers bedeutet,
wobei die Zeolithe Porenweiten von mindestens 5 Å haben,
durchgeführt wird.

2. Verfahren nach Anspruch 1, dadurch gekennzeichnet, daß 0,2-5 Mol Ether, bevorzugt 0,5-2 Mol Ether pro Mol Acylierungsmittel eingesetzt werden.

3. Verfahren nach Anspruch 1, dadurch gekennzeichnet, daß als Acylierungsmittel die Anhydride oder die Säurechloride eingesetzt werden.

4. Verfahren nach Anspruch 1, dadurch gekennzeichnet, daß in Gegenwart von 1-100 Gew.-%, bevorzugt 5-50 Gew.-%, besonders bevorzugt 10-30 Gew.-% Zeolith-Katalysatoren, bezogen auf das Gesamtgewicht der umzusetzenden organischen Reaktionspartner, gearbeitet wird.

5. Verfahren nach Anspruch 1, dadurch gekennzeichnet, daß sich die umzusetzenden organischen Reaktionspartner in der flüssigen Phase befinden.

6. Verfahren nach Anspruch 1, dadurch gekennzeichnet, daß $Me^1$ ein 3-wertiges Element, bevorzugt Aluminium allein, ist.

7. Verfahren nach Anspruch 1, dadurch gekennzeichnet, daß Zeolithe der Strukturen Faujasit, L, Mordenit, Mazzit, Offretit, Gmelinit, Cancrinit, ZSM 12, ZSM 25, Zeolith $\beta$, Ferrierit, ZSM 5, ZSM 11, Heulandit, ZSM 22, ZSM 23, ZSM 48, ZSM 43, ZSM 35, PSH-3, Zeolith $\rho$, ZSM 38, CSZ-1, ZSM 3, ZSM 20, Chabasit, bevorzugt Strukturtypen L, Mordenit, ZSM 5, ZSM 11, ZSM 12, ZSM 23 oder Offretit, besonders bevorzugt Strukturtypen des Mordenit, ZSM 5, L oder ZSM 11, eingesetzt werden.

8. Verfahren nach Anspruch 7, dadurch gekennzeichnet, daß 50 bis 100 %, bevorzugt 80 bis 100 % der austauschbaren Kationen $H^+$-Ionen sind.

9. Verfahren nach Anspruch 8, dadurch gekennzeichnet, daß die $H^+$-Formen von Mordenit, ZSM 5, ZSM 11, L, ZSM 12, ZSM 23 und Offretit eingesetzt werden.